Europäisches Patentamt

European Patent Office

Office européen des brevets

(19)

(11) Publication number: **0 306 168**
**A1**

## (12) EUROPEAN PATENT APPLICATION

(21) Application number: **88307510.3**

(22) Date of filing: **12.08.88**

(51) Int. Cl.⁴: **C07B 59/00 , A61K 43/00 , A61K 49/02 , C07C 159/00**

(30) Priority: **12.08.87 US 84544**

(43) Date of publication of application:
**08.03.89 Bulletin 89/10**

(84) Designated Contracting States:
**AT BE CH DE ES FR GB GR IT LI LU NL SE**

(71) Applicant: IMMUNOMEDICS, INC.
**150 Mt. Bethel Road, CN 4918**
**Warren New Jersey 07060(US)**

(72) Inventor: **Wu, Robert Sundoro**
**25 Ralph Road**
**West Orange New Jersey 07052(US)**

(74) Representative: **Sheard, Andrew Gregory et al**
**Kilburn & Strode 30, John Street**
**London WC1N 2DD(GB)**

(54) Preparation of radiolabeled conjugates.

(57) A method is provided for preparing a radiolabeled conjugate, which comprises the steps of:

(a) contacting an active ester derivative or a thiol-reactive derivative of the bis-thiosemicarbazone of a 1,2-, 1,3- or 1,4-dicarbonyl compound with a radioisotope solution of reduced pertechnetate or perrhenate or of copper, mercury or lead/bismuth ions, to form a radiolabeled bis-thiosemicarbazone chelate without substantially affecting the active ester or the thiol-reactive function; and

(b) reacting the radiolabeled bis-thiosemicarbazone chelate with (i) an amine in the case of an active ester derivative, or (ii) a thiol in the case of a thiol-reactive derivative, to form a radiolabeled conjugate.

Also provided are chelators for radioisotopes, radiolabeling agents for labeling an amine or a thiol, kits and injectable preparations for use therewith, and improved imaging methods for in vivo human use employing the foregoing compositions and methods.

EP 0 306 168 A1

# PREPARATION OF RADIOLABELED CONJUGATES

## BACKGROUND OF THE INVENTION

The present invention relates to methods for preparing radiolabeled conjugates, especially antibody or antibody fragment conjugates, using an efficient prelabeling technique. The methods are especially effective for labeling with technetium, rhenium and copper, in particular Tc-99m, Re-186 and Cu-67. Labeling agents for use in the foregoing methods are also provided.

Technetium-99m is a gamma-emitting radioisotope which has attractive physical properties, e.g., 141 KeV emission and 6 hr half-life, for external gamma scintigraphy and which has the further advantages of low cost and ready availability. The chemistry of Tc-99m is somewhat complex, however, and existing methods for preparing Tc-labeled conjugates have not solved all of the problems in this area. In particular, methods of labeling proteins, especially antibodies or antibody fragments, with Tc-99m have been fraught with difficulty.

Direct labeling of proteins with reduced Tc-99m has been reported. Generally, such methods rely upon production of free thiol groups in the protein by reducing disulfide bonds in the molecule, and the Tc ions are produced by reduction of generator-produced pertechnetate, $TcO_4^-$. The particular valence state and ionic species of technetium resulting from reduction of pertechnetate are not well established, in most cases, and these may well depend on the presence or absence of stabilizing agents, and/or upon the nature of the reducing agent.

Various chelators have been conjugated to proteins and the conjugates have then been reacted with reduced pertechnetate, sometimes in the presence of other stabilizing agents for the reducing agent and/or the reduced form(s) of technetium. Labeling of conjugates of ethylenediaminetetraacetic acid (EDTA), diethylenetriaminepentaacetic acid (DTPA), and varous diamine/dithiol compounds with proteins has been disclosed.

A major disadvantage of known labeling methods is that labeling of an antibody/chelator conjugate generally involves contact of the antibody with an excess of the reducing agent for the pertechnetate, most commonly stannous ion. This often results in reduction of at least some of the disulfide bonds in the antibody, which in turn results in some loss of integrity of the molecule, including a reduction in immunoreactivity. In addition, thiol groups produced by such cleavage of disulfide bonds compete for reduced pertechnetate and result in some labeling which is not in the form of the desired chelate complex. It appears that such thio-bound technetium may be more easily lost to other organs in the body and/or less efficiently excreted than chelated technetium.

A labeling method that introduces reduced pertechnetate into a chelator which contains a functional group for later reaction with a group on an antibody or, more generally, on another compound to which it is desired to attach a technetium label, would have a significant advantage over current methods which label a preformed chelator/antibody conjugate.

Rhenium is found just below technetium in the periodic table, has the same outer shell electronic configuration, and therefore is expected to have very similar chemical properties, especially the behavior of analogous compounds. In fact, rhenium compounds behave similarly to technetium compounds insofar as reduction and chelation are concerned but their greater susceptibility to oxidation requires greater care in handling.

The radioisotope Re-186 is attractive for both imaging and therapy. It has a half-life of about 3.7 days, a high LET beta emission (1.07 MeV) and a convenient gamma emission energy (0.137 MeV). Like technetium, rhenium is produced from perrhenate, and the reduced rhenium ions can bind non-specifically to protein. Accordingly, a method for Re-186 labeling of proteins wherein the reduced perrhenate is first chelated by an activated chelator, and then conjugated to another compound, e.g., a protein molecule such as an antibody, would be advantageous to avoid non-specific labeling and/or degradation of the protein by the reducing system.

Copper ions are also tightly chelated by sulfur-nitrogen chelators. Cu-67 is another attractive radionuclide for imaging and therapy. It has a half-life of about 2.6 days, a beta emission (0.570 MeV) and a gamma emission (0.185 MeV), although the beta energy is relatively low. Cu-67 is relatively expensive and not readily available at present, although such conditions can change as demand develops. It has the advantage that it forms stable chelates, the labeling is simple and rapid, and requires no reducing agent. Non-specific binding can be a problem if it is desired to bind Cu (II) ions to amine and especially to thiol-containing compounds. In such cases, it would be desirable to pre-label a chelator with Cu-67 and then react the chelator with protein to effect conjugation.

Other radionuclides with similar chelation behavior to copper, e.g., mercury and lead, also could be bound to amine and thiol-containing compounds with greater specificity using a pre-labeling technique. Hg-197 has a half-life of about 1.5 days, and emits gamma radiation in an energy range of 78-268 KeV, making it suitable for gamma scintigraphy. Bi-212 is an alpha-emitter with a half-life of about 1 hr and an energy of 6.09 MeV, making it of considerable interest for in vivo therapy. It is produced in situ from a Pb-212 precursor with emission of gamma radiation of 239 KeV, with a half-life of about 10.6 hr. Thus, antibody conjugates for Bi-212 therapy will be Pb-212-labeled conjugates, and the short-hand notation lead/bismuth of Pb/Bi is used herein to indicate this.

A labeling method and reagent for efficient pre-labeling of a chelate that could then react with an amine or thiol-containing compound to form a specifically labeled conujugate would fill a need and solve problems which currently hamper use of certain radionuclides for in vivo imaging and therapy.

## OBJECTS OF THE INVENTION

One object of the present invention is to provide a general method for producing a radiolabeled conjugate, wherein a radioisotope of technetium, rhenium, copper, mercury or lead/bismuth is bound to a bis-thiosemicarbazone function of a chelator capable of later reaction with an amine or a thiol group to form a labeled conjugate.

Another object of the invention is to provide stable technetium, rhenium, mercury and lead/bismuth labeling agents for use in the foregoing method.

A further object of the invention is to provide an improved method of imaging tumors using technetium labeled antibodies or antibody fragments labeled according to the foregoing method.

Yet another object of the invention is to provide an improved method of imaging and/or treating tumors using rhenium-, copper-, mercury- or lead/bismuth-labeled antibodies or antibody fragments labeled according to the foregoing method.

Upon further study of the specification and appended claims, further objects and advantages of this invention will become apparent to those skilled in the art.

## SUMMARY OF THE INVENTION

These objects can be achieved by providing a method for preparing a radiolabeled conjugate, which comprises the steps of:

(a) contacting an active ester derivative or a thiol-reactive derivative of the bis-thiosemicarbazone of a 1,2-, 1,3- or 1,4-dicarbonyl compound with a radioisotope solution of reduced pertechnetate or perrhenate or of copper, mercury or lead/bismuth ions, to form a radiolabeled bis-thiosemicarbazone chelate without substantially affecting the active ester or the thiol-reactive function; and

(b) reacting the radiolabeled bis-thiosemicarbazone chelate with (i) an amine in the case of an active ester derivative, or (ii) a thiol in the case of a thiol-reactive derivative, to form a radiolabeled conjugate.

The invention further provides chelators for radioisotopes, radiolabeling agents for labeling an amine or a thiol, kits and injectable preparations for use therewith, and improved imaging and therapy methods for in vivo human use employing the compositions and methods of the invention.

## DETAILED DESCRIPTION

The labeling agents of the invention incorporate a bis-thiosemicarbazone as a chelating function to bind reduced pertechnetate or perrhenate or to bind copper, mercury or lead/bismuth ions. The bis-thiosemicarbazone is formed by reacting a 1,2-, 1,3- or 1,4-dicarbonyl compound, having ketone or aldehyde carbonyls in close proximity, preferably a 1,2- or 1,3-dicarbonyl compound, more preferably a 1,2-dicarbonyl compound, and most preferably a 1,2-ketoaldehyde (a substituted glyoxal), with thiosemicarbazide or, preferably, an N-alkyl-thiosemicarbazide, most preferably N-methyl-thiosemicarbazide. The dicarbonyl compound will also have a carboxyl group which can be converted to an activated ester for later conjugation to an amine, or it will incorporate a thiol-reactive group for later conjugation to a thiol-containing compound.

Suitable dicarbonyl compound will have the two ketone or aldehyde carbonyls is sufficiently close proximity to permit the bis-thiosemicarbazone to form a stable chelate with reduced pertechnetate or perrhenate, or with copper, mercury or lead/bismuth ions. By "reduced pertechnetate" or "reduced perrhenate" is meant the species of technetium or rhenium ion formed by chemical reduction of pertechnetate or perrhenate and chelated by the bis-thiosemicarbazone. It is generally thought that reduced pertechnetate is in the form of Tc(III) and/or Tc(IV) and/or Tc(V) in such chelates, and that reduced perrhenate is in the form of Re(III) and/or Re(IV) and/or Re(V), but higher or lower oxidation states and/or multiple oxidation states cannot be excluded and are within the scope of the invention. Copper will normally be in the form of Cu(II), although Cu(I) and/or Cu(III) are not excluded. Mercury will normally be in the form of Hg-(I) and/or Hg(II). Lead/bismuth will normally be in the form of Pb(II) or Pb(IV).

The dicarbonyl compound can be any diketone, dialdehyde or ketoaldehyde having proximate carbonyl groups, generally either on adjacent carbon atoms or separated by one, two or more atoms. It will generally be the case that bis-thiosemicarbazones of 1,2- and 1,3-dicarbonyl compounds will form the most stable chelates with reduced pertechnetate and perrhenate ions and with copper, mercury and lead/bismuth ions, although larger chelate rings may be stable, e.g., if their geometries are fixed and the chelating atoms are held close together, e.g., on fused or bridged ring systems. 1,3-dicarbonyl compounds will generally be blocked at the 2-position between the two carbonyl groups by substituents, e.g., dialkyl, an ethylenic or imine linkage , or the like, or will be nonenolizable. Similarly, 1,4-dicarbonyl compounds can react with a single thiosemicarbazide to form a pyrazine if one of the carbonyls is enolizable to a carbon atom between the two carbonyls, and such enolization must be blocked or prevented by proximity to a bridgehead of a bridged ring system.

It will also be appreciated that 1,2-dicarbonyl compounds can be generated in situ from hydroxycarbonyl compounds during the course of reaction with thiosemicarbazide, in an "osazone" type reaction, a reaction well known to sugar chemists. In this reaction, formation of a thiosemicarbazone promotes oxidation of an adjacent hydroxyl to a carbonyl, which then reacts with a second thiosemicarbazide to form the bis-thiosemicarbazone osazone analogue. Alternatively, Cu(II) oxidation of the vicinal hydroxycarbonyl compound to form the 1,2-dione may be employed.

As used herein, the term "stable chelate" means a chelate which, after conjugation to an antibody, retains chelated Tc, Re, Cu, Hg or Pb/Bi atoms to the extent of at least about 80%, preferably at least about 90%, more preferably at least about 95%, after incubation in human serum, for 24 hours, at 37° C. Normally, in such chelates, each of the two thiosemicarbazone groups contributes two coordinating electron pairs to form at least a tetradentate chelate with the metal ion. It will be noted that loss of radiometal ions is conveniently detected by observing the extent of formation of low molecular weight radioactive species, e.g., by size exclusion HPLC using a gamma detector. As a confirming test, injection of radiometal-labeled antibody conjugate into an animal, withdrawal of blood 24 hrs after injection , and passage of the plasma through an affinity column containing bound antigen for the antibody of the conjugate, should show retention of not less than about 80% of the radioactivity on the column, preferably at least about 90%, more preferably at least about 95%.

Another qualitative stability test is resistance of the chelate to transchelation when challenged with a large excess of a multidentate chelator such as ethylenediaminetetraacetic acid (EDTA). Preferably, there is substantially no loss of radiometal from a labeled antibody conjugate when incubated at pH 7.4 (approximating physiological pH) with at least a 100-fold molar excess, preferably a 1000-fold excess, of EDTA for about 0.5 hr, at room temperature, as determined by failure to detect substantially any low molecular weight radioactive Tc, Re, Cu, Hg or Pb/Bi species by HPLC analysis using a size exclusion column.

The dicarbonyl compounds can have other chains, aliphatic and/or aromatic homocyclic or heterocyclic rings, or non-interfering substituents thereon, without limitation. Alkyl, aryl, cycloalkyl, polyoxyalkylene, heterocyclic or other groups, or combinations thereof, can be incorporated into the dicarbonyl compound. Where the labeled chelator is to be used to conjugate to an antibody/fragment or other protein, the conjugation reaction will normally be effected in aqueous solution, under te conditions which do not denature or otherwise compromise the protein's essential structural or functional features. Thus, the chelate is preferably soluble in the aqueous medium.

Generally, 1,2-dicarbonyl compounds are preferred for making bis-thiosemicarbazone chelates. One preferred subclass of 1,2-dicarbonyl compounds comprises substituted glyoxals, i.e., a 1,2-ketoaldehyde joined to other moieties which contain either a carboxyl function for conjugation to an amine or a thiol-reactive derivative for conjugation to a thiol-containing compound. An illustrative examples is p-carboxyethylphenylglyoxal (CEPG), $HOOCCH_2CH_2C_6H_4COCHO$.

It will be appreciated that the phenylene ring and/or the ethylene chain can be replaced by other rings and/or chains, substituted or unsubstituted, straight or branched, or otherwise modified, so long as the moieties do not interfere with the radiometal labeling or preservation of the function used for conjugation.

It will generally be useful, although not necessarily critical, to separate the dicarbonyl function from the ester or thiol-reactive group by at least one, and preferably at least several atoms, to reduce adverse steric interactions between the chelate and the compound to which it is conjugated. Thus, for example, a glyoxal, or other dicarbonyl function, will be joined to a carboxyl or thiol-reactive group through an intermediate spacer moiety, such as a ring and/or chain. It will sometimes be advantageous to incorporate hydrophilic or lipophilic groups in this spacer. The hydrophilic groups can promote uptake of the reduced pertechnetate ions, while the lipophilic groups can promote shielding of the formed chelate by groups on the molecule to which the chelate is conjugated, thereby protecting the chelated metal ions from being transferred to another chelator or removed from the chelate and reoxidized. Hydrophilic and/or lipophilic substituents on the chelator can also affect the ease of conjugation of the labeled chelator to the compound to which it is to be joined, e.g., a protein such as an antibody or antibody fragment, as well as the biodistribution, clearance and excretion of antibody conjugates thereof, although the precise nature of such effects may not be readily predictable.

The general formula for the phenylglyoxal subclass is Q-R-COCHO, wherein Q is an activated ester or a thiol-reactive group, and R is a spacer, which in its simplest form is a single bond, but which can be any combination of carbon chains and/or rings having, e.g., 1-30 carbon atoms, optionally containing heteroatoms, e.g., 0-4 nitrogen and/or oxygen atoms, and optionally bearing non-interfering substituents. It will be appreciated that hydroxycarbonyl equivalents that undergo "osazone" formation, i.e., Q-R-CH(OH)CHO and Q-R-CO-CH$_2$OH, as well as equivalents of the glyoxal or the foregoing ketol/aldol, bearing a substituent, e.g., alkyl, preferably lower alkyl, aryl, aralkyl, or the like, in place of the hydrogen atom on the aldehyde carbon, can all form 1,2-bis-thiosemicarbazones of this general type.

Illustrative examples include those where Q is COOSu, COOphthalimidyl (COOPth), COOAr (Ar is, e.g., C$_6$Cl$_5$, C$_6$F$_5$, C$_6$H$_2$(SO$_3$)$_3$), CONHR'-maleimide (R' is, e.g., (CH$_2$)$_m$, m = 2-20), and the like, optionally substituted, e.g., with hydroxyls; and where R is (CH$_2$)$_n$, n = 1-20, or (CH$_2$)$_q$-Ar-, q = 0-20, and Ar is, e.g., -C$_6$H$_4$- and the like. Preferred compounds are those wherein Q is COOSu, COOPth, COONHCH$_2$CH$_2$-maleimide or COONHCH$_2$CH(OH)CH$_2$-maleimide; and R is (CH$_2$)$_{0-6}$C$_6$H$_4$.

The dicarbonyl compounds can be synthesized by a variety of techniques. Many known compounds will also be usable or adaptable by facile modification to use as chelators. For example, CEPG is a known compound, described by Arano et al., pp. 32-33, Abstracts, 5th Intl. Symp. Radiopharm. Chem., Tokyo, Japan, 1984; Intl. J. Nucl. Med. Biol., 12:425- 430, 1985, and prepared by a straightforward pathway by these authors. Ethyl phenylpropionate was acylated with acetyl chloride under Friedel-Crafts conditions, to give the p-acetyl derivative (also available commercially). After hydrolysis, the acetyl group of the ketoacid was oxidized with selenium dioxide to form the glyoxal, CEPG, which was converted to the bis-N-methylthiosemicarbazone by reaction with N-methylthiosemicarbazide. Analogues with other alkylene groups than ethylene between the phenyl ring and the carboxyl group are readily prepared from the corresponding phenylalkylcarboxylic acids by the same acylation/oxidation sequence. Examples of such analogues, with chain lengths of 0-10 carbon atoms between the phenyl ring and the carboxyl group, have also been disclosed by Arano et al., supra, and at Appl. Radiat. Isot., 37:587-592, 1986.

Other types of spacers can be incorporated in the dicarbonyl compound by conventional synthetic methods. For example, straight or branched chains can be formed with an alcohol at one end and a halide at the other, the halide can be displaced with acetylide, which can then be hydrated to an acetyl group, while the hydroxyl can be oxidized to a carboxylic acid, after which the acetyl group can be oxidized with selenium dioxide to form a glyoxal. Benzene rings can be hydrogenated to form cyclohexane rings, other carbocyclic rings can be formed by, e.g., aldol or Claisen condensation of dicarbonyl compounds, Diels-Alder cyclization or various other well-known cyclization reactions. Heterocyclic rings or chains containing heteroatoms can be introduced by conventional methods, e.g., used of reductive amination, condensation reactions, alkylation, rearrangement reactions and the like, all of which will be within the skill of the ordinary artisan.

The 1,2-ketols and 1,2-aldols are readily prepared by a variety of conventional reactions, e.g., acyloin and benzoin condensations, homologation reactions used in sugar chemistry, e.g., Kiliani-Fischer type synthesis, and the like. Alternatively, hydrolysis of alpha-halo ketones or aldehydes, prepared by, e.g., hydrogen halide opening of epoxides and oxidation of the halohydrin, or acid-catalyzed halogenation of ketones or aldehydes, results in the desired intermediates. Sugars themselves can be functionalized at remote positions to attach the active ester or thiol-reactive functions either prior to or subsequent to "osazone" reaction with a thiosemicarbazide.

Suitable activated esters include, but are not limited to, substituted or unsubstituted succinimidyl (Su), phthalimidyl (Pth) or carbocyclic or heterocyclic aryl (Ar), preferably phenyl, esters, and have the property that they are substantially stable to the conditions used to reduce pertechnetate or perrhenate, in the case of Tc- or Re-labeling, and label the bis-thiosemicarbazone portion of the chelator, but are reactive enough to readily conjugate to an amine group, especially pendant amine groups on the lysine residues of protein molecules, especially antibodies and antibody fragments, without excessive hydrolysis. As used herein, "substantially stable" to reduction and labeling conditions means that at least about 80%, preferably at least about 90%, more preferably at least about 95% of the ester is intact after Tc, Re, Cu, Hg or Pb/Bi labeling of the chelator. As used herein, "readily conjugate" means that the conjugation reaction is substantially complete, i.e., about 90% or more complete, in not more than about 3 hrs, preferably not more than about 2 hrs, more preferably not more than about 1 hr. It must be understood that the yield of conjugate need not be 100% when the reaction is substantially complete, merely that no further reaction occurs. It must also be understood that the conjugation reaction may be quenched prior to completion, e.g., with glycine, to reduce decay of the label and to optimize loading of the compound, e.g., antibody, to which the label is being conjugated and retention of its immunoreactivity. As used herein, "without excessive hydrolysis" means hydrolysis of not more than about 30%, preferably not more than about 20% and more preferably not more than about 5-10% of the ester as a by-product of the conjugation reaction.

Preferred esters are succinimidyl and phthalimidyl esters, either unsubstituted or substituted with one or more solubilizing groups, e.g., sulfonate. Aryl esters will generally have electron withdrawing groups thereon, e.g., one or preferably several sulfonate groups, halogen atoms, e.g., F, Cl, Br, and the like, to increase their rate of acylation by amine groups.

Generally, such esters are prepared from the corresponding acids by reaction with an alcohol or phenol, in the presence of a suitable condensing agent, e.g., dicyclohexylcarbodiimide (DCC) or an analogue thereof. Such condensing agents and conditions for their use are conventional and well known to the ordinary skilled artisan. In subsequent discussions, the activated ester group will be illustrated as a succinimidyl ester (COOSu), but it should be understood that any other ester having the required stability to radiometal labeling conditions and reactivity towards amine conjugation will be suitable for use in the invention.

Suitable thiol-reactive functions are those which are substantially stable to Tc, Re, Cu, Hg or Pb/Bi labeling conditions but which react readily with free sulfhydryl groups. Again, "substantially stable" to reduction and labeling conditions means that at least about 80%, preferably at least about 90%, more preferably at least about 95% of the thiol-reactive group retains its ability to react with thiols after radiometal labeling of the chelator. As used herein, "react readily" means that the conjugation reaction with a thiol-containing compound is substantially complete in not more than about 3 hrs, preferably not more than about 2 hrs, more preferably not more than about 1 hr (again, the yield may be less than 100% at the point where no further reaction occurs, and reaction may be quenched, e.g., by addition of cysteine, prior to completion to optimize loading and retention of immunoreactivity and to reduce radioactive decay).

Preferred functions are substituted or unsubstituted maleimide groups or analogous structures, e.g., maleamides, fumaramides, half esters thereof, or similarly activated ethylenic compounds which react readily with a thiol group to form a thioether. Activating functions other than carbonyl ester, amide or imide groups can include aryl groups, either carbocyclic or heterocyclic, with suitable electron-withdrawing properties, phosphonyl groups, cyano groups, and the like, provided they are also stable to the radiometal labeling conditions. Generally, activated, electron deficient ethylenic compounds that are good dienophiles for dienes such as butadiene will be good thiol-reactive species towards sulfhydryl addition to the ethylenic double bond, and will be good condidates for coupling to the bis-thiosemicarbazone chelator.

An illustrative derivative is $CH(TSC)C(TSC)$-$C_6H_4CH_2CH_2CONHCH_2CH_2$-maleimide. The foregoing compound and its homologues, wherein the ethylidene chain between the benzene ring and the carboxyl can vary from zero to 20 carbons in length, straight or branched, preferably 1-6 carbons, and/or wherein the ethylidene chain between the amide nitrogens can vary from 2 and 20 carbons, preferably 2-6 carbons, are preferred.

Substituents on these thiol-reactive groups can include any groups that do not interfere with the attachment of the group to the chelator, the reaction of the chelator with the radiometal ions or the reaction of the labeled chelator with a thiol-containing molecule, especially a protein with free sulfhydryl groups, and particularly with an antibody or antibody fragment. Such sustituents typically can include hydroxyl and/or alkoxyl, preferably lower alkoxyl, sulfonate, phosphonate and the like.

The thiol-reactive groups can be introduced by reaction of a suitable moiety with the above-described activated esters. As an illustration, reaction of $CH(TSC)C(TSC)C_6H_4CH_2CH_2COOSu$ with $H_2NCH_2CH_2$-maleimide gives the amide-linked derivative, $CH(TSC)C(TSC)$-$C_6H_4CH_2CH_2CONHCH_2CH_2$-maleimide. Variation of the ethylidene chain lengths produces the homologues, and this is simply done by using a longer diamine or a longer chain ester as described above. Similarly, any active ester can be reacted with an amine derivative of any of the thiol-reactive groups mentioned above.

Such derivatives can be obtained, e.g., by reaction of an excess of a straight or branched chain dihalide with a salt of maleimide or an analogue thereof, e.g., the potassium or sodium salt (produced from maleimide or its analogue with, e.g., NaH or $KNH_2$), followed by reaction at the other end of the chain with ammonia or a primary amine, e.g., a lower-alkylamine, aniline or the like. Alternatively, the dihalide can be replaced by a haloalcohol, e.g., 3-bromo-1-propanol (commercially available from Aldrich Chemical Co.) and, after displacement of the halide by the imide salt, the alcohol can be tosylated and displaced by the primary amine. Yet another alternative is reaction of an imide salt with a strained cyclic ether, e.g., an epoxide of 1,3-oxide to generate an alcohol function linked to the imide. Tosylation and reaction with a primary amine again gives the desired function for reaction with an active ester.

The spacer between the bis-thiosemicarbazone and the thiol-reactive function can be substituted by any non-interfering groups, e.g., hydroxyl, alkoxyl, preferably lower alkoxyl, tertiary amine, and the like. It may be desired to link the chelator to a protein or other amine through a spacer containing an ester group, and the foregoing alcohol functions can be joined to a carboxyl on the TSC portion of the chelator by an ester linkage, using DCC or analogous condensing agents.

Alternatively, the thiol-reactive group can be introduced by direct alkylation of an intermediate, condensation, reductive amination, or other conventional reactions familiar to the skilled artisan. Any intermediate that can be displaced by cyanide, to produce a carboxylate after hydrolysis, can be displaced by a maleimide salt, or an amine derivative of the thiol-reactive moiety to produce a thiol-reactive derivative. Other synthetic approaches will be apparent to the ordinary skilled artisan.

Other attractive synthetic pathways to both active ester and thiol-reactive derivatives of bis-thiosemicarbazones of 1,2-dicarbonyls can be envisioned, making use of readily available commercial reagents. The N-hydroxysuccinimide esters and N-hydroxysulfosuccinimide esters of 4-(maleimidylmethyl)cyclohexanecarboxylic acid (SMCC and SMCC-$So_3Na$) and 3-maleimidylbenzoic acid (MBS and MBS-$SO_3Na$) are available as stock reagents, as are sodium D-glucuronate, glucuronamide, glucose pentaacetate, glucopyranose pentabenzoate (all from Aldrich Chemical Co.). These suggest several convenient entries into the 1,2-dicarbonyl derivative subclass.

For example, conversion of glucuronamide into its tetraether, e.g., tetrahydropyranyl (THP) ether by reaction with dihydropyran, or e.g., acetone bisketal by reaction with acetone, followed by hydride reduction, e.g., with lithium aluminum hydride (LAH), reacton of the resultant amine with SMCC, MBS or their sulfonate analogues, hydrolysis of the THP ethers or the ketals, Cu(II) oxidation, and bis-TSC formation gives 6-maleimidyl amides of 6-amino-3,4,5-trihydroxyhexan-1,2-dione bis-N-methylthiosemicarbazone. It will be appreciated that Cu(II) oxidation of glucuronic acid, bis-TSC formation, and esterification with SuOH will give an active ester derivative of this hydroxylated bis-thiosemicarbazone, having greater water solubility for subsequent conjugate formation. Further variations on these pathways will be apparent to the ordinary skilled artisan.

Technetium labeling of the chelator is generally effected by conventional methods. Pertechnetate is obtained from a commercially available generator, most commonly in the form of $NaTcO_4$, normally in saline solution. Other forms of pertechnetate may be used, with appropriate modification of the procedure, as would be suggested by the supplier of a new form of generator or as would be apparent to the ordinary skilled artisan.

Reduction is effected by any of a variety of conventional reducing agents, preferably stannous ion, generally in aqueous solution, optionally with added organic solvent if needed to improve solubility of the chelator. Other suitable reducing agents include, e.g., dithionite, borohydride, ferrous ion and the like.

Pertechnetate is generally used at an activity of about 0,2 -10 mCi/ml in saline, e.g., 0.9% ("physiological") saline, buffered at a pH of about 4 - 7, preferably 5 - 6, usually about 5.5. Suitable buffers include, e.g., actate, tartrate, citrate, phosphate and the like.

When stannous ion is used, it is normally added in excess, to assure complete reduction of utilization of the pertechnetate. The stannous ion concentration is generally in the range of $10^{-4}$ - $10^{-3}$ N, and it is usually added in the form of $SnCl_2$/0.1N HCl.

The chelator is normally present in the reduction reaction mixture, generally in excess, relative to both the reducing agent and the pertechnetate, e.g., in a Sn:chelate molar ratio of about 1:5 -1:250, preferably 1:10 - 1:50, more preferably about 1:25.

The reduction is normally effected under an inert gas atmosphere, e.g., nitrogen, argon or the like. The reaction temperature is generally maintained at about room temperature, e.g., 18 - 25° C, and the reaction usually requires about 0.5 - 3 hrs for substantial completion. It is again noted that the reaction may be termination by quenching prior to completion, for the reasons discussed above.

If an organic solvent is used to improve the solubility of the chelator, it will be preferably a polar, inert solvent, e.g., dimethylformamide (DFM), dimethylsulfoxide (DMSO), methanol, ethanol, acetonitrile or the like water-miscible solvent.

Progress of the reaction can be monitored by spotting an aliquot of the reaction mixture on a silica gel thin layer chromatographic (TLC) strip, and developing the strip in , e.g., 95:5 acetone:acetic acid or 90:10 ethyl acetate:methanol. The labeled chelator is generally detected by a gamma counter at R.f. = 0.8-0.9, unreduced pertechnetate at R.f. = 0.5, and reduced by unchelated Tc (probably as $TcO_2$) at R.f. = 0.

It is often advantageous to add a transchelating agent and/or a stabilizing agent for the stannous ion. It is known that ascorbate can improve specific loading of a chelator with reduced pertechnetate and minimize formation of $TcO_2$, when the reducing agent is stannous ion. Other polycarboxylic acids, e.g., tartrate, citrate, phthalate, iminodiacetate and the like, can also be used. While the precise role of such agents is not known, it appears that they chelate stannous ion and may prevent adventitious reactions and/or promote reduction by stabilization of stannic ions, and they may also chelate -- and thereby stabilize -- certain oxidation states of reduced pertechnetate and perrhenate, thereby serving as transchelating agents for the transfer of these technetium and rhenium ions to the presumably more stable bis-thiosemicarbazone chelator. Such agents will be referred to as "transchelators" herein.

The transchelator should form a chelate with the technetium or rhenium ion that readily and rapidly loses the ion to the nitrogen/sulfur atoms of the bis-thiosemicarbazone. Although polycarboxylic acids are mentioned, by way of illustration, any of a variety of anionic and/or hydroxylic oxygen-containing species could serve this function, e.g., salicylates, acetylacetonates, hydroxyacids, catechols, glycols and other polyols, e.g., glucoheptonate, and the like. The molar ratio of transchelator to chelator should be about 100:1 - 1:1, preferably 50:1 - 20:1, more preferably about 10:1.

After labeling, a scavenger for unreacted reduced pertechnetate, and/or stannous and/or stannic ions can be added to the reaction mixture to prevent reaction of these byproducts with the compound to be conjugated to the chelate. Such scavengers can include, e.g., polycarboxylic acids such as those mentioned above, polyaminocarboxylic acids such as diethylenetriaminepentaacetic acid (DTPA), EDTA, iminodiacetic acid and the like. The scavenger will normally be added in an amount calculated to remove substantially all of the unreacted reduced pertechnetate and/or stannous ions and/or stannic ions.

Another alternative to adding such scavengers is passage of the reduction mixture through a column containing derivatized adsorbants capable of trapping pertechnetate, e.g., gel columns with bound chelators for reduced pertechnetate and/or stannous/stannic ions. For example, a polymer column packing with bound iminodiacetic acid, called "Chelex", is available from BioRad. Other such suitable column packings include polymers or gells modified to contain DTPA EDTA and the like, a number of which are commercially available.

Rhenium labeling will be effected in substantially the same manner as technetium labeling, with special care being taken to insure the absence of air or oxygen in the system. Re-186 is produced in the form of sodium perrhenate by use of a generator analogous to currently available technetium generators.

Copper labeling will be effected by reaction of an activated ester or thiol-reactive derivative of the chelator with a solution of copper ions, normally Cu(II) ions, in the form of a convenient salt, e.g., chloride, citrate, tartrate or the like, either as available or by mixing of, e.g., the chloride with e.g., sodium citrate, tartrate or the like. Cu-67 is currently available as $CuCl_2$ from Oak Ridge National Laboratories, Tennessee, or from Los Alamos National Laboratories, New Mexico.

Other metallic alpha-, beta- and/or gamma-emitting radionuclides which bind preferentially to sulfur/nitrogen chelators, e.g., Hg-197, Pb-212, and which are available as salts, or in forms which can be readily converted to salts, can also be chelated using the chelators of the present invention. The can be used advantageously, as radiolabels for imaging or as radiotherapeutic agents, in the form of antibody/fragment conjugates prepared according to the method of the invention. Chelate formation is effected analogously to Cu-67 chelate formation.

Mercury radioisotopes are normally available as $HgCl_2$ or as $Hg(NO_3)_2$, e.g., from Oak Ridge National Laboratories.

Lead/bismuth radioisotopes are normally available from Argonne National Laboratories in the form of a supported radon generator.

One of the advantages of the chelates of the invention as radiometal labeling agents is that they can be directly reacted with sthe case of the active ester reagents, or thiol-containing compounds, in the case of thiol-reactive reagents, to form labeled antibody conjugates. The reagents used to label the chelator can be scavenged so that they will not cause damage to the compound to be conjugated/labeled with the labeled chelate, in particular, antibodies and antibody fragments.

For example, an antibody or antibody fragment can be reacted directly with the Tc-labeled chelate bearing an active ester to give labeled antibody/fragment with chelates bound to pendant amines on lysine residues therein. The relative concentrations of chelate and antibody/fragment will determine the loading of Tc label. Thus, using the COOSu derivative of (TSC)$_2$CPEG, under the conditions described above, and with a molar ratio of chelator:antibody of 2:1 -20:1, preferably 2:1 - 10:1, more preferably about 5:1, sufficient activity can be achieved without significant loss of antibody immunoreactivity.

Typically, active ester chelators with COOSu as the active ester moiety are reacted with antibody, in an aqueous buffer, at pH 7.5-10, preferably 8-9, more preferably about 8.5, at ambient temperature, i.e., about 18-15°C, for about 0.2-3 hr, preferably 0.2-1 hr, more preferably about 0.5 hr. Antibody concentration is normally about 1-20 mg/ml, preferably 5-10 mg/ml, more preferably about 8 mg/ml.

Antibodies/fragments with free sulfhydryl groups can be directly reacted with chelates bearing thiol-reactive groups to give labeled antibody/fragment bound by thioether linkages. The free sulfhydryl groups can be introduced onto antibodies/fragments by use of Trout's Reagent, i.e., iminothiolane, using conventional conditions, e.g., those disclosed in Blattler et al., Biochem., 24:1517-1524, 1985.

Alternatively, reaction of the thiol-reactive chelates with Fab' fragments, having free sulfhydryl groups resulting from cleavage of divalent F(ab')$_2$ fragments, will also produce radiometal-labeled conjugates, with the further advantage that the site of reaction will be remote from the binding site of the fragment. Reduction can be effected with appropriate disulfide reducing agents, e.g., cysteine, dithiothreitol, 2-mercaptoethanol, dithionite, SnCl$_2$ or the like, to form the Fab' fragments. Use of a pre-labeled chelate for this reaction minimizes non-specific reaction of the radiometal and/or reducing agent with thiol groups.

Typically, the ratio of thiol-reactive chelate to sulfhydryl groups on the compound to be labeled, e.g., an antibody/fragment, will be 1:10 - 10:1, preferably 1:3 - 3:1, more preferably about 1:2-1:1. The reaction is normally run in aqueous buffer, at pH 5-9, preferably 6-8, more preferably about 7 (using phosphate, carbonate, citrate, borate and like buffers or buffers such as Hepes and the like), at ambient temperature, for about 0.2-3 hr, preferably 0.2-1 hr, more preferably about 0.5 hr, at about the same antibody/fragment concentration as for the active ester coupling.

The reaction is conveniently followed by TLC, using, e.g., 50:50 methanol:ammonium acetate or ammonium citrate. Labeled protein remains at the origin, while unbound chelator migrates with R.f. = 0.9-1.0. Counting of the two regions of the strip will permit an estimate of the extent of reaction.

By prelabeling the chelator with Tc, Re, Cu, Hg or Pb/Bi prior to contact with the sulfhydryl groups of the antibody/fragment, non-specific labeling by attachment of these ions to sulfhydryls is effectively minimized, generally to the point where substantially all of the metal ions are bound to chelator and not to thiols. A scavenger for free sulfhydryl groups can be added to the reaction mixture after conjugation of chelate to the antibody/fragment is substantially complete. Suitable such scavengers include, e.g., maleimide, iodoacetamide, iodoacetic acid and the like. After inital determination of the extent of conjugation of chelator to the thiol-containing compound, an amount of scavenger capable of reacting with substantially all of the remaining sulfhydryl groups is used.

The excess of such sulfhydryl group scavengers can be further scavenged, if desired, by e.g., cysteine or similar such thiol compound, to render them more soluble and thus more readily excretable, so that the conjugation reaction mixture can be used directly as an injectable preparation.

Antibodies and antibody fragments which can be labeled by the foregoing methods include, but are not limited to, antibodies/fragments that specifically bind to antigens which are produced by or associated with tumors, infectious disease-producing organisms, pathological conditions such as myocardial infarction or thrombi in humans or animals, normal organs and tissues, or other antigens of interest for in vivo detection. It will be appreciated that other proteins, e.g., hormones, enzymes, white blood cells and the like, and/or other amine-containing or thiol-containing compounds can also be labeled by the present methods.

Examples of antibodies and antibody fragments which specifically bind markers produced by or associated with tumors, infectious lesions or normal organs and tissues have been disclosed, inter alia, in Hansen et al., U.S. Patent 3,927,193 and Goldenberg, U.S. Patents 4,331,647, 4,348,376, 4,361,544, 4,468,457, 4,444,744, 4,460,459, 4,460,561 and 4,624,846, and in related pending applications EP-A-0208531 and WO-A-8402983, the disclosures of all of which are incorporated herein in their entireties by reference. Antibodies that bind to cardiac myosin are disclosed in Habr, U.S. Patent 4,036,945, for use in imaging myocardial infarction. Antibodies for imaging thrombi and that bind to any of fibrinogen, fibrin, thrombin, platelets, platelet factor or other components or associated antigens of the thrombus may be used for preparing imaging agents for thrombi. Suitable such antibodies/fragments are disclosed inter alia in, e.g., Rosebrough et al., Radiology, 156(2):515-517, 1985; Peters et al., Br. Med. J. (Clin. Res), 293-(6561):1525-1527, 1986; and Ezekowitz et al., Int. J. Rad. Appl. Instrumen., 13(4):407-411, 1986.

The antibody may be whole IgG, IgA, IgD, IgE, IgM or a fragment such as, e.g., F(ab')$_2$, Fab', Fab, monovalent light/heavy chain or the like, including isotypes and subtypes thereof. It can be a polyclonal antibody, preferably an affinity-purified antibody from a human or an appropriate animal, e.g., a goat, rabbit, mouse or the like, or a monoclonal antibody prepared by conventional techniques, e.g., a murine antibody derived from a hybridoma produced by fusion of lymph or spleen cells from a mouse immunized against a lymphatic system antigen with myeloma cells from an appropriate immortal cell line.

It will be appreciated that any other type of antibody/fragment, whether produced by currently known methodology, including chimeric antibodies, hybrids, polyoma and like immunological techniques, or by recombinant DNA-mediated synthesis and expression, cassette-modification, or like techniques, can be labeled by the method of the present invention so long as it bears pendant amine or sulfhydryl functions and the appropriate amine-reactive or thiol-reactive form of the chelate of the invention is used for the conjugation step.

The Tc labeling agents of the invention can be used for preparing antibody/fragment conjugates useful for in vivo imaging of cancer, myocardial infarction, thrombi, abscesses and other infectious diseases and the like, wherever specific antibodies can be used as targeting vehicles for a radiolabel. The labeling method of the invention is an improvement over existing methods of producing Tc-labeled antibody/fragment conjugates in that a stable chelate is prepared prior to contact with the antibody/fragment and an efficient conjugation of the chelate is then effected without contact of the antibody/fragment with unchelated technetium ions. Problems of non-specific labeling, and attendant loss of the label to other organs and tissues, are thereby avoided or minimized.

Hg-labeled antibodies/fragments can be used for imaging since Hg-197 is a gamma emitter in the 50-500KeV range.

The Re, Cu and Pb/Bi labeling agents of the invention can be used for preparing antibody/fragment conjugations that are useful for therapy of cancer and certain infectious lesions that are refractory to chemotherapy, especially deep-seated abscesses. Because Cu-67, Re-186 and Pb-212 are also gamma emitters, they can be used for imaging as a convenient monitor for therapy. Bi-212-conjugates are administered in the form of Pb-212-conjugates which decay to Bi-212-conjugates in situ by high energy gamma emission and then decay rapidly with emission of therapeutically effective alpha radiation. Again, non-specific labeling is minimized or substantially avoided, and problems related to antibody degradation by contact with the chelator labeling reagents, especially reducing agents, are substantially avoided.

The bis-thiosemicarbazone chelators of the invention, in the form of active ester or thiol-reactive derivatives, are stable for extended periods of time when stored in dry, sealed vials under an inert gas atmosphere. They can be supplied in such form as part of radiolabeling kits for use in preparing radiolabeled conjugates. These kits will normally be "cold kits" for use with radioisotope sources at the user location, e.g., technetium generators. Thus, they will contain, in suitable containers for use in a labeling procedure: the chelator; the conjugate partner, e.g., a protein such as an antibody or antibody fragment, generally in lyophylized form; and optionally accessory reagents such as phosphate-buffered saline; a reducing system, e.g., $SnCl_2 \cdot HCl$, optionally with added transchelator/scavenger, e.g., one or more of ascorbate, tartrate, citrate, iminodiacetate; scavengers for unconjugated chelates; or a sizing column for removal of low molecular weight byproducts of conjugate formation. These advantageously will all be in sterile, pyrogen-free sealed containers, generally equipped with syringe septa. Columns may be provided with syringe fittings and/or be incorporated in syringes for case of manipulation.

Alternatively, for certain radioisotopes, sterile, pyrogen-free solutions of labeled conjugate may be supplied by, e.g., a radiopharmacy, to the end user for in vivo use in imaging or therapy.

Without further elaboration, it is believed that one skilled in the art can, using the preceding description, utilize the present invention to its fullest extent. The following preferred specific embodiments are, therefore, to be construed as merely illustrative, and not limitative of the remainder of the disclosure in any way whatsoever. In the following examples, all temperatures are set forth uncorrected in degrees Celsius; unless otherwise indicated, all parts and percentages are by weight.

## EXAMPLE 1

### Synthesis of TSC-4-OSu Active Ester Chelator

A 100 mg sample of 4-carboxyethylphenylglyoxal bis-N-methylthiosemicarbazone (TSC-4-OH), prepared according to the procedure of Arano et al., Intl. J. Nucl. Med. Biol., 12:425-430, 1985, is dissolved in 5 ml of dry dimethylformamide (DMF). Equimolar amounts of N-hydroxysuccinimide (HOSu, 30.2 mg) and dicyclohexylcarbodiimide (DCC, 54.4 mg) are added, under an inert gas atmosphere, and the mixture is stirred for 3 days at room temperature. The mixture is then filtered into metal-free vials and stored under argon in the freezer. Thawed solution is used for subsequent steps.

An aliquot of the solution of activated ester (TSC-4-OSu) is evaporated to dryness, triturated with anhydrous ether, and the resultant powder is vacuum dried, to give a solid, m.p. 79° (softens), 84-90° (decomp.), with MW (MS) 477, $C_{19}H_{23}N_7O_4S_2$.

### EXAMPLE 2

#### Preparation of Tc-99m-labeled Conjugate

To a solution of 100 ug TSC-4-OSu, prepared according to Example 1, in 100 ul DMF, is added 3.9 ul of a solution of 5 ug $SnCl_2 \cdot 6H_2O$ in 0.1 N HCl, with mixing. Then, 100 ul of a solution of 5 mCi of $NaTc-99mO_4$ in saline is added, mixed and incubated for 15-30 minutes. The resultant Tc-99m-labeled chelate is quenched with 1 mg iminodiacetic acid (IDA) in 20 ul of distilled water, and let stand 5 min. A 3 mg portion of anti-CSAp F-(ab')₂ antibody fragment in 596 ul of phosphate-buffered saline (PBS), pH 8.4, is added, and the solution is incubated for 15-30 min, at room temperature, with stirring. HPLC analysis of the resultant solution shows that about 34% of the label is incorporated in the antibody fragment. After separation on preparative HPLC, a fraction containing 72 uCi of Tc-99m, representing 6% recovery of labeled conjugate, is re-analyzed by HPLC, showing essentially 100% purity. Analysis on an anti-CSAp affinity column shows 86% immunoreactivity (>90% recovery), and uv analysis shows about 1.8 chelators/fragment. Additional fractions containing somewhat less pure conjugate are isolated and combined, to give about 19% total recovery of conjugate. Challenge of a sample of the labeled conjugate with a 100-fold excess of EDTA resulted in >95% retention of the label.

Addition of a 10-fold molar excess of sodium ascorbate in place of the IDA, relative to the TSC-4-OSu, during the pre-labeling step also facilitates the incorporation. Purification of the reaction mixture by passage through a short sizing gel column removes substantially all of the unconjugated radiometal.

Substantially the same procedure is used to prepare Re-186-labeled conjugates.

### EXAMPLE 3

#### Preparation of TSC-4-maleimide

TSC-4-OSu, prepared according to Example 2, is reacted with excess 6-aminohexylmaleimide to give the TSC-4-maleimide derivative (TSC-4-M).

### EXAMPLE 4

#### Preparation of Re-186-labeled Conjugate

Reaction of the TSC-4-M derivative, prepared according to the procedure of Example 3, with 186-perrhenate and $SnCl_2$ according to the procedure of Example 2 (with TSC-4-OSu replaced by TSC-4-M) gives Re-186-TSC-4-M. Reaction of baboon anti-CEA antiserum with Trout's reagent results in introduction of an average of 2 free sulfhydryl groups per antibody molecule. Incubation of the labeled chelate with the thiolated baboon anti-CEA antiserum, in PBS containing excess ascorbate, at pH 7.2, in substantially the same proportions as in Example 2, gives the Re-186-labeled conjugate in good yield after about 1 hr incubation. Immunoreactivity is substantially the same as for the intact antiserum.

### EXAMPLE 5

#### Preparation of Cu-67-labeled Conjugate

A solution of TSC-4-M, prepared according to the procedure of Example 3, is reacted with 67-$CuCl_2$, in the presence of ascorbate, to give the Cu-67-labeled chelate. Incubation of the Cu-67-TSC-4-M with thiolated baboon anti-CEA antiserum, according to the procedure of Example 4, gives Cu-67-labeled conjugate without substantial loss of immunoreactivity.

Substantially similar conditions are used for labeling with $Hg(NO_3)_2$ and with $PbCl_2$.

### EXAMPLE 6

## Tumor Localization

patient having a rising CEA titer, and having a history of a prior colon tumor resection after successful imaging with radio-labeled anti-CSAp F-(ab')₂, is imaged with a sterile, pyrogen-free solution of the Tc-99m-labeled anti-CSAp F(ab')₂ prepared according to Example 2. The solution is prepared and administered analogously to Examples 5 and 6 of Goldenberg U.S. Patent 4,331,657, with the evident difference that Lugol's solution is unnecessary when no subtraction is done. Imaging is effected both with and without subtraction. Subtraction is done with I-131-labeled normal goat F-(ab')₂. Early imaging of a recurrence of the colonic tumor is achieved with subtraction after 18 hours, with improved resolution after 36 hours. Lung and liver metastases are seen, the lung metastates being detectable without subtraction after 8-12 hours. It is seen that at least some colorectal tumors that secrete CEA are succesfully imaged with anti-CSAp antibodies as well as with anti-CEA antibodies.

### EXAMPLE 7

## Tumor Therapy

The patient showing recurrence of a colonic tumor, with lung metastates, described in Example 6, is treated with a sterile, pyrogen-free solution of the Re-186-labeled anti-CEA baboon antiserum prepared according to Example 4. Unit doses of 200 mCi are administered by intravenous infusion, with successive doses at biweekly intervals until a total of 800 mCi are delivered. Significant reduction in colon tumor volume and apparent substantial disappearance of liver and lung metastases are achieved, as shown by imaging at periodic intervals. The therapeutic solution and mode of administration analogous to those in Example 7 of Goldenberg U.S. Patent 4,348,376.

The preceding examples can be repeated with similar success by substituting the generically or specifically described reactants and/or operating conditions of this invention for those used in the preceding examples.

From the foregoing description, one skilled in the art can easily ascertain the essential characteristics of this invention and, without departing from the spirit and scope thereof, can make varous changes and modifications of the invention to adapt it to various usages and conditions.

## Claims

1. A method for preparing a radiolabeled conjugate, which comprises the steps of:

(a) contacting an active ester derivative or a thiol-reactive derivative of the bis-thiosemicarbazone of a 1,2-, 1,3- or 1,4-dicarbonyl compound with a radioisotope solution of reduced pertechnetate or perrhenate or of copper, mercury or lead/bismuth ions, to form a radiolabeled bis-thiosemicarbazone chelate without substantially affecting the active ester or the thiol-reactive function; and

(b) reacting the radiolabeled bis-thiosemicarbazone chelate with (i) an amine in the case of an active ester derivative, or (ii) a thiol in the case of a thiol-reactive derivative, to form a radiolabeled conjugate.

2. The method of Claim 1, wherein said derivative is an active ester derivative, and said radiolabeled bis-thiosemicarbazone chelate is reacted with an amine in step (b).

3. The method of Claim 2, wherein said activated ester is an N-hydroxysuccinimide (OSu) or N-hydroxyphthalimide (OPth) ester.

4. The method of Claim 2, wherein said amine is an antibody or an antibody fragment containing lysine residues having reactive amine functions thereon.

5. The method of Claim 1, wherein said derivative is a thiol-reactive derivative, and said radiolabeled bis-thiosemicarbazone chelate is reacted with a thiol in step (b).

6. The method of Claim 5, wherein said thiol-reactive derivative is a maleimide.

7. The method of Claim 6, wherein said thiol compound is an antibody of antibody fragment containing free sulfhydryl groups.

8. The method of Claim 1, wherein said dicarbonyl compound is a 1,2-dicarbonyl compound.

9. The method of Claim 8, wherein said derivative is a phenylglyoxal-(alkyl)carboxylate bis-N-methylthiosemicarbazone, having the formula Q-R-C₆H₄-C(TSC)-CH(TSC), wherein R is a substituted or unsubstituted, straight or branched chain alkylene group of 1-30 carbon atoms and 0-4 nitrogen and/or oxygen atoms, or a single bond; TSC is N-methylthiosemicarbazone; and Q is an active ester moiety or a thiol-reactive moiety.

10. The method of Claim 9, wherein R is (CH₂)ₙ, and n = 0-6, and wherein Q is a N-hydroxysuccinimide or N-hydroxyphthalimide ester, ZOOC, Z being Su or Pth.

11. The method of Claim 9, wherein R is (CH₂)ₙ, and n = 0-6, and wherein Q is a thiol-reactive moiety containing a maleimide group.

12. The method of Claim 11, wherein Q is CONHR'-maleimide, and R' is $(CH_2)_{2-6}$ or $CH_2CH(OH)CH_2$.

13. The method of Claim 6, wherein said derivative is a 4-(maleimidylmethyl)-cyclohexanecarboxamide or 3-maleimidylbenzamide of 6-amino-3,4,5-trihydroxyhexan-1,2-dione bis-N-methylthiosemicarbazone.

14. The method of Claim 1, wherein said radioisotope is a radioisotope of technetium or rhenium, and wherein said reduced pertechnetate or perrhenate is reduced with stannous ion.

15. The method of Claim 14, wherein said solution of reduced pertechnetate or perrhenate further contains a transchelator, said transchelator being ascorbate, tartrate, citrate or iminodiacetate.

16. The method of Claim 1, wherein said radioisotope is a radioisotope of copper, mercury or lead/bismuth.

17. A method for preparing a radioisotope chelate, which comprises the step of contacting an active ester derivative or a thiol-reactive derivative of the bis-thiosemicarbazone of 1,2-, 1,3- or 1,4-dicarbonyl compound with a radioisotope solution of reduced pertechnetate or perrhenate or of copper, mercury or lead/bismuth ions, to form a radiolabeled bis-thiosemicarbazone chelate, without substantially affecting the active ester or the thiol-reactive function.

18. A method for preparing a radiolabeled conjugate, which comprises the step of reacting a radiolabeled active ester derivative or thiol-reactive derivative of the bis-thiosemicarbazone of a 1,2-, 1,3- or 1,4-dicarbonyl compound, said derivative forming a chelate with a radioisotope of technetium, rhenium, copper, mercury or lead-bismuth, with (i) an amine, in the case of an active ester derivative, or (ii) a thiol, in the case of a thiol-reactive derivative, to form a radiolabeled conjugate.

19. A chelator for radioisotopes which is an active ester derivative or a thiol-reactive derivative of the bis-thiosemicarbazone of a 1,2- or 1,3- or 1,4-dicarbonyl compound.

20. A radiolabeling agent for labeling an amine or a thiol, which comprises an active ester derivative or a thiol-reactive derivative of the bis-thiosemicarbazone of a 1,2-, 1,3- or 1,4-dicarbonyl compound, said derivative forming a chelate with a radioisotope of technetium, rhenium, copper, mercury or lead/bismuth.

21. A radiolabeled conjugate prepared by the method of any of Claims 1, 4, 7 or 18.

22. A kit for preparing a radiolabeled antibody or antibody fragment conjugate for in vivo human use, which comprises, in suitable sterile containers:

(a) a chelator comprising an active ester derivative or a thiol-reactive derivative of the bis-thiosemicarbazone of a 1,2-, 1,3- or 1,4-dicarbonyl compound, said chelator being capable of forming a chelate with a radioisotope of technetium, rhenium, copper, mercury or lead/ bismuth; and

(b) an antibody or antibody fragment which specifically binds an antigen which is produced by or associated with a tumor, infectious lesion, myocardial infarction or thrombus.

23. A sterile, injectable preparation for in vivo human use, which comprises a solution of the radiosotope-labeled antibody/fragment conjugate prepared by the method of any of Claims 4, 7 or 18, in a sterile, pharmaceutically acceptable injection vehicle.

24. A sterile, injectable preparation of Claim 23, wherein said radiolabeled antibody/fragment conjugate is a technetium-99m-labeled antibody/fragment conjugate,

for use in a method of imaging a tumor, infectious lesion, myocardial infarction or thrombus in a patient, which comprises parenterally injecting a human with an antibody or antibody fragment which specifically binds an antigen produced by or associated with a tumor, infectious lesion, myocardial infarction or thrombus, and radio- labeled with a radioisotope capable of external detection by a scanning gamma camera; and scanning with said camera to detect the site or sites of accretion of the labeled antibody/fragment.

25. A sterile, injectable preparation of Claim 23, wherein said radiolabeled antibody/fragment conjugate is a rhenium-186-, copper-67- or lead/bismuth-212-labeled antibody/fragment conjugate,

for use in a method of radioimmunotherapy of a patient having a tumor or a refractory infectious lesion, comprising parenterally injecting a human with an antibody or antibody fragment which specifically binds to an antigen produced by or associated with a tumor or infectious lesion, and radiolabeled with a therapeutically effective amount of a therapeutic radioisotope.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | THE JOURNAL OF NUCLEAR MEDICINE, vol. 28, no. 6, June 1987, pages 1027-1033, New York, US; Y. ARANO et al.: "Technetium-99m-labeled monoclonal antibody with preserved immunoreactivity and high in vivo stability" * Whole article * | 1-4,8-11,14-16 | C 07 B 59/00<br>A 61 K 43/00<br>A 61 K 49/02<br>C 07 C 159/00 |
| A | IDEM | 17-25 | |
| Y | EP-A-0 188 256 (NEORX) * Examples 7,8 * | 1-4,8-11,14-16 | |
| A | EP-A-0 024 464 (NIHON MEDI-PHYSICS) * Claims; example 4 * | 19 | |
| A | EP-A-0 054 920 (NIHON MEDI-PHYSICS) * Claims * | 19 | |

TECHNICAL FIELDS SEARCHED (Int. Cl.4)

C 07 B 59/00
A 61 K 43/00
A 61 K 49/00
C 07 C 159/00

The present search report has been drawn up for all claims

| Place of search | Date of completion of the search | Examiner |
|---|---|---|
| THE HAGUE | 06-12-1988 | WRIGHT M.W. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)